(19) Europäisches Patentamt
European Patent Office
Office européen des brevets

(11) Veröffentlichungsnummer: **0 156 284**
**B1**

(12) # EUROPÄISCHE PATENTSCHRIFT

(45) Veröffentlichungstag der Patentschrift: **10.05.89**

(21) Anmeldenummer: **85103150.0**

(22) Anmeldetag: **19.03.85**

(51) Int. Cl.[4]: **C 07 J 21/00,** C 07 J 53/00, C 07 J 71/00, A 61 K 31/565, A 61 K 31/585

(54) **17-Substituierte Estradiene.**

(30) Priorität: **21.03.84 DE 3410880**

(43) Veröffentlichungstag der Anmeldung: **02.10.85 Patentblatt 85/40**

(45) Bekanntmachung des Hinweises auf die Patenterteilung: **10.05.89 Patentblatt 89/19**

(84) Benannte Vertragsstaaten: **AT BE CH DE FR GB IT LI LU NL SE**

(56) Entgegenhaltungen:
**EP-A-0 019 690**
**FR-A-2 377 418**
**FR-A-2 391 224**
**US-A-3 462 426**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Nikisch, Klaus, Dr., Alt Lichtenrade 11, D-1000 Berlin 49 (DE)**
Erfinder: **Annen, Klaus, Dr., Osthofstrasse 80, D-4400 Münster- Albachten (DE)**
Erfinder: **Laurent, Henry, Dr., Glambecker Weg 21, D-1000 Berlin 28 (DE)**
Erfinder: **Wiechert, Rudolf, Prof. Dr., Petzower Strasse 8 A, D-1000 Berlin 39 (DE)**
Erfinder: **Beiers, Sybille, Dr., Uhlandstrasse 121, D-1000 Berlin 31 (DE)**
Erfinder: **Elger, Walter, Dr., Schorlemer Allee 12 B, D-1000 Berlin 33 (DE)**

**EP 0 156 284 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft 17-substituierte Estradiene der allgemeinen Formel I

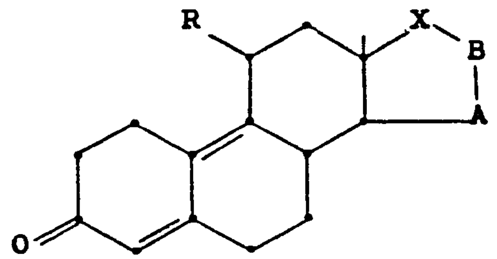

(I),

worin

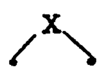 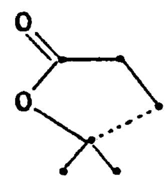, 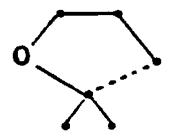, 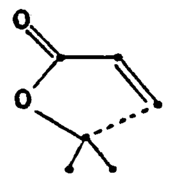, 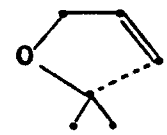,

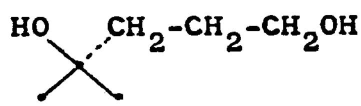 oder 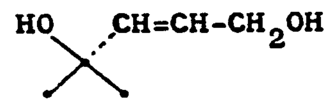,

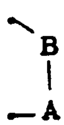 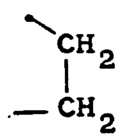 oder 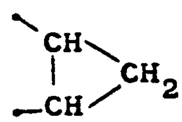

und

R Alkyl, Alkenyl oder Cycloalkyl mit bis zu 5 Kohlenstoffatomen

darstellen, sowie Verfahren zu deren Herstellung und diese enthaltende pharmazeutische Präparate.

Die neuen Verbindungen der allgemeinen Formel I weisen neben einer hohen aldosteronantagonistischen Wirkung eine starke gestagene Potenz auf. Da die neuen Verbindungen das progesteronartige Wirkungsprofil besitzen, werden bei ihrer Verwendung als Kontrazeptiva die sonst auftretenden Nebenwirkungen, wie Blutdruckanstieg und Ödeme, nicht in Erscheinung treten.

Aus den US-Patentschriften 3 509 135, 3 238 197 und 3 764 596 sind Estradiene bekannt, die keine Substituenten in 11- und/oder 15,16-Stellung enthalten. Diese Verbindungen besitzen aldosteronantagonistische und gestagene Wirkung.

Aus der französischen Patentschrift 1 453 222 sind in 11- und 15,16-Stellung unsubstituierte Estratriene mit Antialdosteronwirkung bekannt.

Teilstrukturen, wie der Lactonring unter Einschluß von $C_{17}$, werden in der US-Patentschrift 3 462 426 oder, wie die 3-Keto-4,9-dien-Struktur, in der französischen Offenlegungsschrift 2 377 418 beschrieben. Eine Kombination dieser Teilstrukturen wird durch diese Schriften nicht nahegelegt.

Es wurde gefunden, daß die neuen in 11-Stellung substituierten Verbindungen der allgemeinen Formel I den bisher bekannten Verbindungen dieser Stoffklasse überlegen sind. Der 11β-Alkyl-, Alkenyl- oder Cycloalkylsubstituent bewirkt eine Steigerung der gestagenen und die 15,16-Methylengruppe eine Verstärkung der aldosteronantagonistischen Wirkung.

Im Test auf Antialdosteronwirkung erweisen sich die neuen Verbindungen als dem bekannten Spironolacton gleichwertig.

Im Gestagen-Rezeptor-Bindungstest auf gestagene Wirkung unter Verwendung von Cytosol aus Kaninchenuterushomogenat und von $^3H$-Progesteron als Bezugssubstanz zeigen die neuen Verbindungen eine starke Affinität zum Gestagenrezeptor und sind im Clauberg-Test nach subkutaner Applikation stark wirksam.

In der folgenden Tabelle werden die Kompetitionsfaktoren (K) im Gestagen-Rezeptor-Bindungstest

angegeben. Der Kompetitionsfaktor K als Maß für die Bindungsstärke ist definiert als das Verhältnis der Konzentration der Prüfsubstanz, zur Konzentration des Standards (Progesteron), bei der beide Verbindungen eine gleich große Verdrängung von $^3H$-Progesteron vom Progesteron-Rezeptorkomplex zeigen, so daß ein niedriger K-Wert große Bindungsstärke (hohe Affinität) anzeigt:

**Tabelle**

Gestagen-Rezeptor-Bindungstest

| Verbindung | K (gestagen) |
|---|---|
| A | 0,5 |
| B | 1,6 |
| C | 0,4 |
| D | 1,0 |

A: 19-Nor-3-oxo-11β-vinyl-17α-pregna-4,9-dien-21,17-carbolacton.
B: 17β-Hydroxy-17α-(3-hydroxypropyl)-11β-methyl-4,9-estradien-3-on.
C: 11β-Methyl-19-nor-3-oxo-17α-pregna-4,9-dien-21,17-carbolacton.
D: 11β-Ethyl-19-nor-3-oxo-17α-pregna-4,9-dien-21,17-carbolacton.

Die neuen Verbindungen der allgemeinen Formel I können allein oder in Kombination mit Östrogen in Präparaten zur Kontrazeption verwendet werden. Erfindungsgemäß sollen die neuen Verbindungen besonders bei Frauen eingesetzt werden, die eine Kontrazeption wünschen und bei denen ein Blutdruckanstieg aufgrund vorhandener Risikofaktoren, wie fortgeschrittenes Alter, Übergewicht oder Rauchen, wahrscheinlich ist. Aufgrund des dem natürlichen Progesteron vergleichbaren Profils dieser Substanzen sollte auch bei Frauen, die nicht als Risikopatientinnen eingestuft werden können, das subjektive Wohlbefinden und die Verträglichkeit gegenüber bekannten Präparaten erhöht werden.

Die Dosierung der erfindungsgemäßen Verbindungen in Kontrazeptionspräparaten soll vorzugsweise 0,5 bis 5 mg pro Tag betragen.

Die gestagenen und östrogenen Wirkstoffkomponenten werden in Kontrazeptionspräparaten vorzugsweise zusammen oral appliziert. Die tägliche Dosis wird vorzugsweise einmalig verabfolgt.

Das Östrogen wird in einer Menge verabfolgt, die der von 0,03 bis 0,05 mg Ethinylöstradiol entspricht.

Die neuen Verbindungen der allgemeinen Formel I können auch in Präparaten zur Behandlung gynäkologischer Störungen eingesetzt werden. Wegen ihres günstigen Wirkungsprofils sind die erfindungsgemäßen Verbindungen besonders gut geeignet zur Behandlung praemenstrueller Beschwerden, wie Kopfschmerzen, depressive Verstimmungen, Wasserretention und Mastodynie. Die Tagesdosis bei der Behandlung praemenstrueller Beschwerden liegt bei etwa 1 bis 20 mg.

Die Formulierung der pharmazeutischen Präparate auf Basis der neuen Verbindungen erfolgt in an sich bekannter Weise, indem man den Wirkstoff, gegebenenfalls in Kombination mit einem Östrogen, mit den in der Galenik gebräuchlichen Trägersubstanzen, Verdünnungsmitteln, gegebenenfalls Geschmackskorrigentien usw. verarbeitet und in die gewünschte Applikationsform überführt.

Für die bevorzugte orale Applikation kommen insbesondere Tabletten, Dragees, Kapseln, Pillen, Suspensionen oder Lösungen infrage.

Für die parenterale Applikation sind insbesondere ölige Lösungen, wie zum Beispiel Lösungen in Sesamöl, Rizinusöl und Baumwollsamenöl geeignet. Zur Erhöhung der Löslichkeit können Lösungsvermittler, wie zum Beispiel Benzylbenzoat oder Benzylalkohol, zugesetzt werden.

Die neuen 17-substituierten Estradiene der allgemeinen Formel I enthalten in 11-Stellung eine Alkyl-, Alkenyl- oder Cycloalkylgruppe mit bis zu 5 Kohlenstoffatomen.

Unter Alkyl sollen beispielsweise Methyl, Ethyl, Propyl, Isopropyl, Butyl und Pentyl, unter Alkenyl beispielsweise Vinyl und 2-Propenyl, unter Cycloalkyl beispielsweise Cyclopropyl, Cyclobutyl und Cyclopentyl verstanden werden, bevorzugt sind die Methyl-, Ethyl- und Vinylgruppe.

Die neuen Verbindungen der allgemeinen Formel I werden erfindungsgemäß dadurch hergestellt, daß man in an sich bekannter Weise

a) eine Verbindung der allgemeinen Formel II

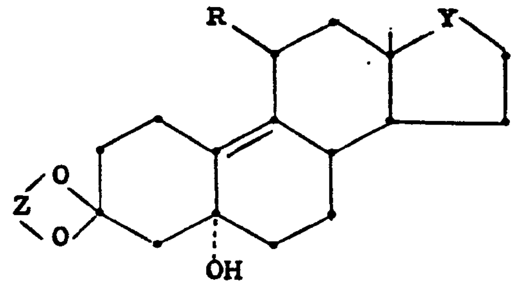

(II),

worin
R die in Formel I angegebene Bedeutung hat,
Y für X steht, wobei vorhandene freie Hydroxygruppen mit einer sauer leicht abspaltbaren Gruppe verethert sein können und
Z eine Ethylen- oder 2,2-Dimethylpropylengruppe
darstellt,

zur Ketalspaltung, Entfernung einer gegebenenfalls vorhandenen mit Säure abspaltbaren Schutzgruppe und gleichzeitigen Wasserabspaltung unter Ausbildung des 4,9-Dien-3-on-Systems mit verdünnter Säure, dem Pyridiniumsalz einer starken Säure oder einem sauren Ionenaustauscher behandelt und gegebenenfalls die so erhaltene 17α-(3-Hydroxypropyl)- oder 17α-(3-Hydroxypropenyl)-Verbindung zur 17-Spiro-Hydrofuran-Verbindung cyclisiert oder zum 21,17-Carbolacton oxydiert oder

b) eine Verbindung der allgemeinen Formel III

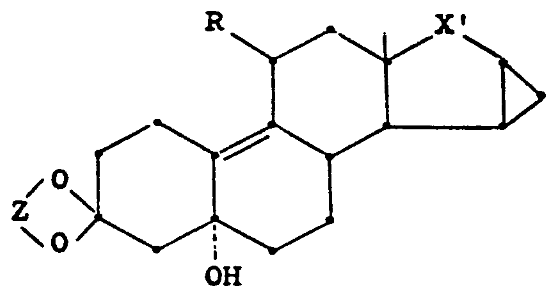

(III),

worin
R die in Formel I angegebene Bedeutung hat,
X'

X'

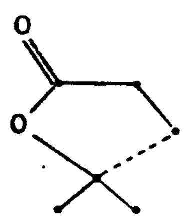
,
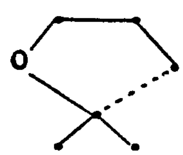
,
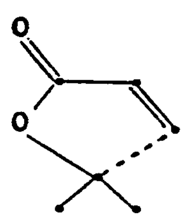
oder
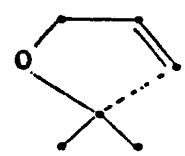

und
Z eine Ethylen- oder 2,2-Dimethylpropylengruppe
darstellen,

zur Ketalspaltung und gleichzeitigen Wasserabspaltung unter Ausbildung des 4,9-Dien-3-on-Systems mit verdünnter Säure, dem Pyridiniumsalz einer starken Säure oder einem sauren Ionenaustauscher behandelt und gegebenenfalls das so erhaltene 21,17-Carbolacton zur 17α-(3-Hydroxypropyl)- oder 17α-(3-Hydroxypropenyl)-Verbindung reduziert, die mitreduzierte 3-Ketogruppe mit Mangandioxid reoxydiert oder

c) eine Verbindung der allgemeinen Formel IV

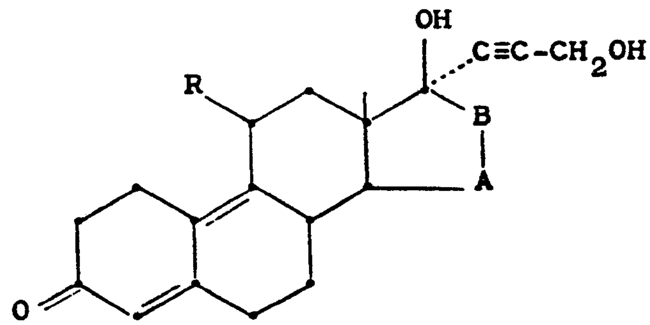

(IV),

worin $\begin{matrix}B\\|\\A\end{matrix}$ und R die in Formel I angegebene Bedeutung haben, zur 17α-(3-Hydroxypropenyl)-Verbindung hydriert bzw. reduziert, diese gegebenenfalls zur entsprechenden 17-Spiro-Hydrofuran-Verbindung cyclisiert oder zum entsprechenden 21,17-Carbolacton oxydiert.

Zur Ketalspaltung, Entfernung einer gegebenenfalls in 22-Stellung vorhandenen Hydroxyschutzgruppe und Wasserabspaltung zum 4,9-Dien-3-Keton wird eine Verbindung der allgemeinen Formel II oder III mit Säure, dem Pyridiniumsalz einer starken Säure oder einem sauren Ionenaustauscher behandelt.

Die in saurem Milieu leicht abspaltbare Hydroxyschutzgruppe ist zum Beispiel eine Methoxymethyl-, Ethoxymethyl- oder Tetrahydropyranylgruppe.

Die saure Behandlung erfolgt in an sich bekannter Weise, indem man eine Verbindung der allgemeinen Formel II oder III in einem mit Wasser mischbaren Lösungsmittel, wie wäßrigem Methanol, Ethanol oder Aceton, löst und auf die Lösung katalytische Mengen Mineralsäure oder Sulfonsäure, wie Salzsäure, Schwefelsäure, Perchlorsäure oder p-Toluolsulfonsäure, so lange einwirken läßt, bis die Ketal- und Wasserabspaltung und die Abspaltung gegebenenfalls vorhandener säureempfindlicher Schutzgruppen beendet sind. Die Umsetzung, die bei Temperaturen von etwa 20 bis 100°C abläuft, kann in besonders hoher Ausbeute auch mit einem Pyridiniumsalz wie Pyridiniumtosylat oder mit einem sauren Ionenaustauscher vorgenommen werden.

Der Verlauf der Umsetzung wird zweckmäßigerweise mit analytischen Methoden, beispielsweise durch Dünnschichtchromatographie entnommener Proben, verfolgt.

Eine nach Verfahren a) oder c) erhaltene 17α-(3-Hydroxypropyl)- oder 17α-(3-Hydroxypropenyl)-Verbindung der allgemeinen Formel I kann anschließend in an sich bekannter Weise zur entsprechenden 17-Spiro-Hydrofuran-Verbindung der allgemeinen Formel I cyclisiert oder zum entsprechenden 21,17-Carbolacton der allgemeinen Formel I oxydiert werden.

Die Oxydation wird mit den üblichen Oxydationsmitteln, wie zum Beispiel Jones-Reagenz, Chromsäure-Pyridin, Pyridiniumdichromat oder Pyridiniumchlorochromat, vorgenommen.

Die Cyclisierung erfolgt mit wasserabspaltenden Mitteln, beispielsweise mit p-Toluolsulfonsäurechlorid in Gegenwart von Pyridin.

Ein nach Verfahren b) erhaltenes 21,17-Carbolacton der allgemeinen Formel I kann anschließend in an sich bekannter Weise zur 17α-(3-Hydroxypropyl)- oder 17α-(3-Hydroxypropenyl)-Verbindung der allgemeinen Formel I reduziert werden. Zur Reduktion sind beispielsweise Metallhydride wie Lithiumaluminiumhydrid geeignet. Die mitreduzierte 3-Ketogruppe kann anschließend mit Mangandioxid reoxydiert werden.

Nach Verfahren c) wird die Dreifachbindung der 17α-(3-Hydroxypropinyl)-Verbindung der allgemeinen Formel IV zur Z- bzw. E-konfigurierten Doppelbindung hydriert bzw. reduziert.

Die Verbindung mit der Z-konfigurierten Doppelbindung entsteht durch Hydrieren der acetylenischen Dreifachbindung in Gegenwart eines desaktivierten Edelmetallkatalysators (J. Fried, J.A. Edwards: Organic Reactions in Steroid Chemistry, Van Nostrand Reinhold Company 1972, Seite 134, und H.O. House: Modern Synthetic Reactions 1972, Seite 19). Als desaktivierte Edelmetallkatalysatoren kommen beispielsweise 10 % Palladium auf Bariumsulfat in Gegenwart eines Amins oder 5 % Palladium auf Calciumcarbonat unter Zusatz von Blei(II)-acetat nach Lindlar infrage. Die Hydrierung wird nach der Aufnahme von einem Äquivalent Wasserstoff abgebrochen.

Die Verbindung mit der E-konfigurierten Doppelbindung entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem Soc. 63 (1941) 216), mit Natriumamid in flüssigem Ammoniak (J. Chem. Soc. 1955, 3558), mit Lithium in niedermolekularen Aminen (J. Am. Chem. Soc. 77 (1955) 3378) mit Boranen (J. Am. Chem. Soc. 93 (1971) 3395 und 94 (1971) 6560), mit Diisobutyl-aluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245).

Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxidationsstufe.

**Herstellung der Ausgangsverbindungen der allgemeinen Formel II**

**Vorschrift 1**

Zu einer Lösung von 25 g Propagylalkohol-tetrahydropyranylether in 500 ml Tetrahydrofuran (THF) tropft man unter Argon 238 mmol n-Butyllithium und rührt 15 Minuten nach. Dazu tropft man 16,6 g 3,3-(2,2-Dimethylpropylen-1,3-dioxy)-5α,10α-epoxy-9(11)-estren-17-on in 300 ml THF und rührt 30 Minuten nach. Anschließend fällt man in 3,5 l Eiswasser, extrahiert mit Essigester und wäscht mit Wasser neutral. Nach Chromatographie an Aluminiumoxid erhält man 26 g 3,3-(2,2-Dimethyltrimethylendioxid)-5α,10α-epoxy-17α-(tetrahydropyranyloxypropinyl)-9(11)-estren-17β-ol.

Eine Lösung von 15,4 g 3,3-(2,2-Dimethyltrimethylendioxy)-5α,10α-epoxy-17α-tetrehydropyranyloxypropinyl)-9(11)-estren-17β-ol in 200 ml THF wird mit 3 g Tristriphenylphosphin-Rhodium-I-chlorid hydriert. Nach Aufnahme von 1350 Wasserstoff wird im Vakuum eingeengt. Das erhaltene Rohprodukt wird an Aluminiumoxid chromatographiert. Man erhält 13,8 g 3,3-(2,2-Dimethyltrimethylendioxid)-5α,10α-epoxy-17α-(tetrahydropyranyloxy)-propyl-9(11)-estren-17β-ol.

Zu 95 ml einer 1,3 molaren Vinylmagnesiumbromidlösung gibt man bei -30°C 150 mg $Cu_2Cl_2$ und rührt 30 Minuten bei dieser Temperatur. Dazu tropft man eine Lösung von 8,5 g 3,3-(2,2-Dimethyltrimethylendioxy)-5α,10α-epoxy-17α-(tetrahydropyranyloxy)-propyl-9(11)-estren-17β-ol in 100 ml THF und rührt 2 Stunden bei -20°C nach. Anschließend gießt man in kalte Ammonchloridlösung, extrahiert mit Ether, wäscht mit Wasser, trocknet über Natriumsulfat und engt im Vakuum ein. Das erhaltene Rohprodukt wird an Aluminiumoxid chromatographiert. Man erhält 6,5 g 3,3-(2,2-Dimethyltrimethylendioxy)-11β-vinyl-17α-(3-tetrahyropyranyloxy)-propyl-9-estren-5α,17β-diol.

**Vorschrift 2**

Zu einer aus 1,3 g Magnesium und 6,57 g 2-Brompropen in 100 ml THF hergestellten Grignardlösung gibt man bei -30°C 100 mg $Cu_2Cl_2$ und rührt 30 Minuten nach. Dazu tropft man eine Lösung von 2,78 g 3,3-(2,2-Dimethyltrimethylendioxy)-5α,10α-epoxy-17α-[(3-tetrahydropyranyloxy)-propyl]-9(11)-estren-17β-ol in 35 ml THF und rührt 3 Stunden nach, wobei die Temperatur bis auf 0°C ansteigt. Anschließend gießt man in Ammonchloridlösung, extrahiert mit Ether, wäscht mit Wasser, trocknet und engt im Vakuum ein. Das erhaltene Rohprodukt wird an Aluminiumoxid chromatographiert. Man erhält 2,4 g 3,3-(2,2-Dimethyltrimethylendioxy)-11β-isopropenyl-17α-(3-tetrahydropyranyloxy)-propyl-9-estren-5α,17β-diol.

**Vorschrift 3**

Zu einer aus 7,07 g Magnesium und 18,2 ml Methyliodid in 440 ml Ether hergestellten Grignardlösung gibt man bei -30°C 150 mg Kupfer-I-chlorid und rührt 30 Minuten bei dieser Temperatur nach. Dazu tropft man eine Lösung von 10 g 3,3-(2,2-Dimethyltrimethylendioxy)-5α,10α-epoxy-17α-[(3-tetrahydropyranyloxy)-propyl]-9(11)-estren-17β-ol in 100 ml Ether und rührt 30 Minuten bei dieser Temperatur nach. Anschließend gießt man in Ammonchloridlösung, extrahiert mit Ether, wäscht mit Wasser und trocknet über Natriumsulfat. Das erhaltene Rohprodukt wird an Aluminiumoxid chromatographiert. Man erhält 7,8 g 3,3-(2,2-Dimethyltrimethylendioxy)-11β-methyl-17α-[(3-tetrahydropyranyloxy)-propyl]-9-estren-5α,17β-diol.

**Vorschrift 4**

Unter den in Vorschrift 3 beschriebenen Bedingungen erhält man aus 5 g 3,3-(2,2-Dimethyltrimethylendioxy)-5α,10α-epoxy-17α-[(3-tetrahydropyranyloxy)-propyl]-9(11)-estren-17β-ol und Ethylmagnesiumbromid 3,4 g 3,3-(2,2-Dimethyltrimethylendioxy)-11β-ethyl-17α-[(3-tetrahydropyranyloxy)-propyl]-9-estren-5α,17β-diol.

**Vorschrift 5**

Unter den in Vorschrift 3 beschriebenen Bedingungen erhält man aus 4 g 3,3-(2,2-Dimethyltrimethylendioxy)-5α,10α-epoxy-17α-[(3-tetrahydropyranyloxy)-propyl]-9(11)-estren-17β-ol und Propylmagnesiumbromid 2,2 g 3,3-(2,2-Dimethyltrimethylendioxy)-11β-propyl-17α-[(3-tetrahydropyranyloxy)-propyl]-9-estren-5α,17β-diol.

**Vorschrift 6**

Unter den in Vorschrift 3 beschriebenen Bedingungen erhält man aus 4,5 g 3,3-(2,2-Dimethyltrimethylendioxy)-5α,10α-epoxy-17α-[(3-tetrahydropyranyloxy)-propyl]-9(11)-estren-17β-ol und Cyclopropylmagnesiumbromid 2,9 g 11β-Cyclopropyl-3,3-(2,2-dimethyltrimethylendioxy)-17α-[(3-tetrahydropyranyloxy)-propyl]-9-estren-5α,17β-diol.

**Vorschrift 7**

Unter den in Vorschrift 3 beschriebenen Bedingungen erhält man aus 1,2 g 3,3-(2,2-Dimethyltrimethylendioxy-5α,10α-epoxy-17α-[(3-tetrahydropyranyloxy)-propyl]-9(11)-estren-17β-ol 750 mg 11β-Cyclopentyl-3,3-(2,2-dimethyltrimethylendioxy)-17α-[(3-tetrahydropyranyloxy)-propyl]-9-estren-5α, 17β-diol.

**Vorschrift 8**

a) Unter den in Vorschrift 3 beschriebenen Bedingungen erhält man aus 2,5 g 3,3-(2,2-Dimethyltrimethylendioxy)-5α,10α-epoxy-9(11)-estren-17-on 1,5 g 11β-Cyclopropyl-3,3-(2,2-dimethyltrimethylendioxy)-5α-hydroxy-9-estren-17-on.
b) Unter den in Vorschrift 1 beschriebenen Bedingungen erhält man aus 1 g 11β-Cyclopropyl-3,3-(2,2-dimethyltrimethylendioxy)-5α-hydroxy-9-estren-17-on 1,3 g 11β-Cyclopropyl-3,3-(2,2-dimethyltrimethylendioxy)-17-[3-(tetrahydropyranyloxy)-1-propinyl]-9-estren-5α,17β-diol.
c) Eine Lösung von 1,2 g 11β-Cyclopropyl-3,3-(2,2-dimethyltrimethylendioxy)-17-[3-(tetrahydropyranyloxy)-1-propinyl]-9-estren-5α,17β-diol in 12 ml wasserfreiem Ethanol wird mit 60 mg Lindlar-Katalysator 5 Stunden bei Raumtemperatur hydriert. Nach dem Abfiltrieren des Katalysators engt man ein und reinigt das erhaltene Rohprodukt durch Chromatographie an Kieselgel. Man erhält 750 mg 11β-Cyclopropyl-3,3-(2,2-dimethyltrimethylendioxy)-17-[-3-(tetrahydropyranyloxy]-1-(Z)-propenyl-9-estren-5α,17β-diol.

**Herstellung der Ausgangsverbindungen der allgemeinen Formel III**

**Vorschrift 9**

a) Zu einer Lösung von 20 g 15α-Hydroxy-estra-4,9-dien-3,17-dion in 120 ml Pyridin tropft man 13,6 ml Benzoylchlorid und rührt eine Stunde bei Raumtemperatur nach. Anschließend gießt man in Eiswasser, extrahiert mit Dichloridmethan, wäscht mit Natriumbicarbonatlösung und Wasser und engt im Vakuum ein. Das erkaltete Rohprodukt wird an Kieselgel chromatographiert. Man erhält 26,2 g 15α-Benzoyloxy-estra-4,9-dien-3,17-dion.
b) Eine Lösung von 8,7 g Trimethylsulfoxoniumiodid in 200 ml Dimethylsulfoxid (DMSO) wird mit 1,625 g 55 %-igem Natriumhydrid eine Stunde lang gerührt. Dazu gibt man 4,9 g 15α-Benzoyloxy-estra-4,9-dien-3,17-dion, rührt 10 Minuten und gießt dann in Eiswasser ein. Anschließend extrahiert man die wäßrige Phase mit Dichlormethan und reinigt das erhaltene Rohprodukt durch Säulenchromatographie an Kieselgel. Man erhält 2,07 g 15β,16β-Methylenestra-4,9-dien-3,17-dion.
IR: 1710, 1660, 1580 $cm^1$
UV: $\varepsilon_{302} = 20.000$
c) Eine Lösung von 10 g 15β,16β-Methylen-estra-4,9-dien-3,17-dion in 25 ml Dichlormethan wird mit 10 g 2,2-Dimethylpropandiol-1,3 12,7 ml o-Ameisensäuretriethylester und 125 g p-Toluolsulfonsäure 20 Minuten bei Raumtemperatur gerührt. Anschließend versetzt man mit Triethylamin und wäscht mit Wasser. Nach dem Eindampfen wird das erhaltene Rohprodukt an Kieselgel chromatogrphiert. Man erhält 8,20 g 3,3-(2,2-Dimethyltrimethylendioxy)-15β,16β-methylen-estra-5(10),9(11)-dien-17-on vom Schmelzpunkt 159° C.
IR: 1710 $cm^{-1}$
UV: $\varepsilon_{242} = 18.500$
$[\alpha]_D = +223°$
d) Eine Lösung von 32,2 g 3,3-(2,2-Dimethyltrimethylendioxy)-15β,16β-methylen-estra-5(10),9(11)-dien-17-on in 200 ml Dichlormethan wird unter Eiskühlung nacheinander mit 1,26 ml Hexachloraceton und 14,5 ml 30 %-igem Wasserstoffperoxid versetzt und 24 Stunden bei Raumtemperatur gerührt. Anschließend wird mit Natriumthiosulfatlösung und Wasser gewaschen, getrocknet und eingeengt. Das erhaltene Rohprodukt wird an Aluminiumoxid chromatographiert. Man erhält 17,5 g 3,3-(2,2-Dimethyltrimethylendioxy)-5α,10α-epoxy-15β,16β-methylen-estr-9(11)-en-17-on vom Schmelzpunkt 173,4° C.

e) Eine Lösung von 11,4 g 3,3-(2,2-Dimethyltrimethylendioxy)-5α,10α-epoxy-15β,16β-methylen-estr-9(11)-en-17-on in 200 ml Tetrahydrofuran (THF) wird unter Eiskühlung mit 45,4 g Kaliumethylat und 17 ml Propagylalkohol versetzt und 30 Minuten gerührt. Anschließend gießt man in Eiswasser, neutralisiert mit Schwefelsäure, extrahiert mit Essigester, wäscht mit Wasser und engt im Vakuum ein. Man erhält 12,1 g 3,3-(2,2-Dimethyltrimethylendioxy)-5α,10α-epoxy-15β,16β-methylen-17α-(3-hydroxypropinyl)-estr-9(11)-en-17β-ol.

f) Eine Lösung von 11,9 g 3,3-(2,2-Dimethyltrimethylendioxy)-5α,10α-epoxy-15β,16β-methylen-17α-(3-hydroxypropinyl)-estr-9(11)-en-17β-ol in 175 ml THF wird mit 2,5 g Tristriphenylphosphin-Rhodium-I-chlorid hydriert. Nach Aufnahme von 1090 ml Wasserstoff wird im Vakuum eingeengt und an Aluminiumoxid chromatographiert. Man erhält 9,7 g 3,3-(2,2-Dimethyltrimethylendioxy)-5α,10α-epoxy-15β,16β-methylen-17α-(3-hydroxypropyl)-estr-9(11)-en-17β-ol.

g) Unter den in Vorschrift 3 beschriebenen Bedingungen erhält man aus 6 g 3,3-(2,2-Dimethyltrimethylendioxy)-5α,10α-epoxy-15β,16β-methylen-17α-(3-hydroxypropyl)-estr-9(11)-en-17β-ol 3,9 g 3,3-(2,2-Dimethyltrimethylendioxy)-11β-methyl-15β,16β-methylen-17α-(3-hydroxypropyl)-estr-9-en-5α,17β-diol.

h) Unter den in Beispiel 3 b) angegebenen Bedingungen erhält man aus 3,8 g 3,3-(2,2-Dimethyltrimethylendioxy)-11β-methyl-15β,16β-methylen-17α-(3-hydroxypropyl)-estr-9-en-5α,17β-diol 2,8 g 3,3-(2,2-Dimethyltrimethylendioxy)-5α-hydroxy-11β-methyl-15β,16β-methylen-19-nor-17α-pregn-9-en-21,17-carbolacton.
IR: 3350, 1760 $cm^{-1}$.

**Vorschrift 10**

a) Unter den in Vorschrift 3 angegebenen Bedingungen erhält man aus 5 g 3,3-(2,2-Dimethyltrimethylendioxy)-5α,10α-epoxy-15β,16β-methylen-17α-(3-hydroxypropyl)-estr-9(11)-en-17β-ol 2,7 g 3,3-(2,2-Dimethyltrimethylendioxy)-5α-hydroxy-15β,16β-methylen-11β-vinyl-17α-(3-hydroxypropyl)-estr-9-en-5α,17β-diol.

b) Unter den in Beispiel 3 b) angegebenen Bedingungen ethält man aus 2,6 g 3,3-(2,2-Dimethyltrimethylendioxy)-5α-hydroxy-15β,16β-methylen-11β-vinyl-17α-(3-hydroxypropyl)-estr-9-en-5α,17β-diol 1,9 g 3,3-(2,2-Dimethyltrimethylendioxy)-5α-hydroxy-15β,16β-methylen-11β-vinyl-19-nor-17α-pregn-9-en-21,17-carbolacton.
IR: 3350, 1760 $cm^{-1}$.

**Vorschrift 11**

a) Unter den in Vorschrift 3 beschriebenen Bedingungen erhält man aus 4,5 g 3,3-(2,2-Dimethyltrimethylendioxy)-5α,10α-epoxy-15β,16β-methylen-17α-(3-hydroxypropyl)-estr-9(11)-en-17β-ol 3,15 g 11β-Cyclopropyl-3,3-(2,2-dimethyltrimethylendioxy)-15β,16β-methylen-17α-(3-hydroxypropyl)-estr-9-en-5α,17β-diol.

b) Unter den in Beispiel 3 b) angegebenen Bedingungen erhält man aus 3,1 g 11β-Cyclopropyl-3,3-(2,2-dimethyltrimethylendioxy)-15β,16β-methylen-17α-(3-hydroxypropyl)-estr-9-en-5α,17β-diol 1,9 g 11β-Cyclopropyl-3,3-(2,2-dimethyltrimethylendioxy)-5α-hydroxy-15β,16β-methylen-19-nor-17α-pregn-9-en-21,17-carbolacton.
IR: 3350, 1760 $cm^{-1}$.

Auf analoge Weise werden die Ausgangsverbindungen der allgemeinen Formeln IV hergestellt.

**Beispiel 1**

a) Zu einer Losung von 6,5 g 3,3-(2,2-Dimethyltrimethylendioxy)-11β-vinyl-17α-[(3-tetrahydropyranyloxy)-propyl]-9-estren-5α,17β-diol in 200 ml 90 %-igem Ethanol gibt man 50 mg p-Toluolsulfonsäure und rührt 45 Minuten bei 75°C. Anschließend gießt man in Wasser ein, stellt mit Ammoniak alkalisch und extrahiert mit Dichlormethan. Nach dem Waschen und Trocknen engt man im Vakuum ein. Das erhaltene Rohprodukt wird an Kieselgel chromatographiert. Man erhält 3,2 g 17β-Hydroxy-17α-(3-hydroxypropyl)-11β-vinyl-4,9-estradien-3-on.
IR: 3400, 1660, 1620 $cm^{-1}$.
UV: $\varepsilon_{301} = 14.200$

b) Zu einer Lösung von 3,2 g 17β-Hydroxy-17α-(3-hydroxypropyl)-11β-vinyl-4,9-estradien-3-on in 170 ml Aceton tropft man bei -10°C 16 ml Jones-Lösung und rührt 30 Minuten bei dieser Temperatur nach. Anschließend wird mit Methanol das überschüssige Reagenz zerstört, mit Essigester verdünnt und mit Wasser gewaschen. Nach dem Trocknen und Einengen wird das erhaltene Rohprodukt an Kieselgel chromatographiert. Man erhält 1,56 g 19-Nor-3-oxo-11β-vinyl-17α-pregna-4,9-dien-21,17-carbolacton.
Schmelzpunkt: 133 - 135°C.

**Beispiel 2**

a) Zu einer Lösung von 2,2 g 3,3-(2,2-Dimethyltrimethylendioxy)-11β-isopropenyl-17α-[3-(tetrahydropyranyloxy)-propyl]-9-estren-5α,17β-diol in 70 ml 90 %-igem wäßrigen Ethanol gibt man 20 mg p-Toluolsulfonsäure und erhitzt 30 Minuten auf 80°C. Anschließend gießt man in Eiswasser, stellt mit Ammoniak alkalisch und extrahiert mit Dichlormethan. Nach dem Waschen und Trocknen engt man im Vakuum ein. Das erhaltene Rohprodukt wird an Kieselgel chromatographiert. Man erhält 920 mg 17β-Hydroxy-17α-(3-hydroxypropyl)-11β-isopropenyl-4,9-estradien-3-on.
$[\alpha]_D$ = -70°

b) Zu einer Lösung von 620 mg 17β-Hydroxy-17α-(3-hydroxypropyl)-11β-isopropenyl-4,9-estradien-3-on in 35 ml Aceton tropft man bei -10°C 3,6 ml Jones-Lösung und rührt 30 Minuten bei dieser Temperatur nach. Anschließend versetzt man mit wenig Methanol, verdünnt mit Essigester und wäscht mit Wasser. Nach dem Trocknen über Natriumsulfat und Einengen wird das erhaltene Rohprodukt an Kieselgel chromatographiert. Man erhält 375 mg 19-Nor-3-oxo-11β-isopropenyl-17α-pregna-4,9-dien-21,17-carbolacton.
$[\alpha]_D$ = -38°

**Beispiel 3**

a) Zu einer Lösung von 7,7 g 3,3-(2,2-Dimethyltrimethylendioxy)-11β-methyl-17α-[(3-tetrahydropyranyloxy)-propyl]-9-estren-5α,17β-diol in 300 ml 90 %-igem wäßrigen Ethanol gibt man 0,1 g p-Toluolsulfonsäure und rührt 40 Minuten bei 75°C. Anschließend gießt man in Eiswasser ein, stellt mit Ammoniak alkalisch und extrahiert mit Dichlormethan. Nach dem Waschen mit Wasser und Trocknen über Natriumsulfat engt man im Vakuum ein. Das erhaltene Rohprodukt wird an Kieselgel chromatographiert. Man erhält 4,85 g 17β-Hydroxy-17α-(3-hydroxypropyl)-11β-methyl-4,9-estradien-3-on.
$[\alpha]_D$ = -109,1°

b) Zu einer Lösung von 4,8 g 17β-Hydroxy-17α-(3-hydroxypropyl)-11β-methyl-4,9-estradien-3-on in 270 ml Aceton gibt man bei -70°C 27 ml Jones-Lösung und rührt 30 Minuten bei dieser Temperatur nach. Anschließend versetzt man mit wenig Methanol und engt im Vakuum weitgehend ein, verdünnt mit Essigester, wäscht mit Wasser, trocknet über Natriumsulfat und engt im Vakuum ein. Das erhaltene Rohprodukt wird an Kieselgel chromatographiert. Man erhält 1,6 g 11β-Methyl-19-nor-3-oxo-17α-pregna-4,9-dien-21,17-carbolacton.
Schmelzpunkt: 198 - 201°C.

**Beispiel 4**

a) Unter den in Beispiel 3 a) beschriebenen Bedingungen erhält man aus 3,3 g 3,3-(2,2-Dimethyltrimethylendioxy)-11β-ethyl-17α-[(3-tetrahydropyranyloxy)-propyl]-9-estren-5α,17β-diol 1,45 g 11β-Ethyl-17β-hydroxy-17α-(3-hydroxypropyl)-4,9-estradien-3-on.

b) Unter den in Beispiel 3 b) beschriebenen Bedingungen erhält man aus 1,35 g 11β-Ethyl-17β-hydroxy-17α-(3-hydroxypropyl)-4,9-estradien-3-on 620 mg 11β-Ethyl-19-nor-3-oxo-17α-pregna-4,9-dien-21,17-carbolacton.
$[\alpha]_D$ = -94,1°

**Beispiel 5**

a) Unter den in Beispiel 3 a) beschriebenen Bedingungen erhält man aus 2,1 g 3,3-(2,2-Dimethyltrimethylendioxy)-11β-propyl-17α-[(3-tetrahydropyranyloxy)-propyl]-9-estren-5α,17β-diol 1,2 g 17β-Hydroxy-17α-(3-hydroxypropyl)-11β-propyl-4,9-estradien-3-on.
$[\alpha]_D$ = -52,3°

b) Unter den in Beispiel 3 b) beschriebenen Bedingungen erhält man aus 1 g 17β-Hydroxy-17α-(3-hydroxypropyl)-11β-propyl-4,9-estradien-3-on 520 mg 19-Nor-11β-propyl-3-oxo-17α-pregna-4,9-dien-21,17-carbolacton.
$[\alpha]_D$ = 70°

**Beispiel 6**

a) Unter den in Beispiel 3 a) beschriebenen Bedingungen erhält man aus 2,8 g 11β-Cyclopropyl-3,3-(2,2-dimethyltrimethylendioxy)-17α-[(3-tetrahydropyranyloxy)-propyl]-9-estren-5α,17β-diol 1,45 g 11β-Cyclopropyl-17β-hydroxy-17α-(3-hydroxypropyl)-4,9-estradien-3-on.

b) Unter den in Beispiel 3 b) beschriebenen Bedingungen erhält man aus 1,35 g 11β-Cyclopropyl-17β-hydroxy-17α-(3-hydroxypropyl)-4,9-estradien-3-on 870 mg 11β-Cyclopropyl-19-nor-3-oxo-17α-pregna-4,9-dien-21,17-carbolacton.
Schmelzpunkt: 156 - 158° C.

**Beispiel 7**

a) Unter den in Beispiel 3 a) beschriebenen Bedingungen erhält man aus 700 mg 11β-Cyclopentyl-3,3-(2,2-dimethyltrimethylendioxy)-17α-[(3-tetrahydropyranyloxy)-propyl]-9-estren-5α,17β-diol 312 mg 11β-Cyclopentyl-17β-hydroxy-17α-(3-hydroxypropyl)-4,9-estradien-3-on.
$[\alpha]_D$ = -101,6°

b) Unter den in Beispiel 3 b) beschriebenen Bedingungen erhält man aus 270 mg 11β-Cyclopentyl-17β-hydroxy-17α-(3-hydroxypropyl)-4,9-estradien-3-on 154 mg 11β-Cyclopentyl-17β-hydroxy-19-nor-3-oxo-17α-pregna-4,9-dien-21,17-carbolacton.
$[\alpha]_D$ = -38°

**Beispiel 8**

Eine Lösung von 695 mg 11β-Cyclopropyl-3,3-(2,2-dimethyltrimethylendioxy)-17α-[3-(tetrahydropyranyloxy)-1-(Z)-propenyl-9-estren-5α,17β-diol in 11 ml Ethanol wird mit 71 mg Pyridiniumtosylat 30 Minuten bei 55° C gerührt. Anschließend verdünnt man mit Dichlormethan, wäscht mit Natriumhydrogencarbonatlösung und Wasser und engt im Vakuum ein. Das erhaltene Rohprodukt wird an Kieselgel chromatographiert. Man erhält 470 mg 11β-Cyclopropyl-17-hydroxy-17α-(3-hydroxy-1-Z-propenyl)-4,9-estradien-3-on als Schaum.
$[\alpha]_D$ = -69,1°

**Beispiel 9**

Unter den in Beispiel 3 angegebenen Bedingungen erhält man aus 2,7 g 3,3-(2,2-Dimethyltrimethylendioxy)-5α-hydroxy-11β-methyl-15β,16β-methylen-19-nor-17α-pregn-9-en-21,17-carbolacton 1,2 g 11β-Methyl-15β,16β-methylen-3-oxo-19-nor-17α-pregna-4,9-dien-21,17-carbolacton.
IR: 1760, 1660 $cm^{-1}$
UV: $\varepsilon_{301}$ = 20.050

**Beispiel 10**

Unter den in Beispiel 3 angegebenen Bedingungen erhält man aus 1,9 g 3,3-(2,2-Dimethyltrimethylendioxy-5α-hydroxy-15β,16β-methylen-11β-vinyl-19-nor-17α-pregn-9-en-21,17-carbolacton 870 mg 15β,16β-Methylen-3-oxo-11β-vinyl-19-nor-17α-pregna-4,9-dien-21,17-carbolacton.
IR: 1760, 1660 $cm^{-1}$
UV: $\varepsilon_{300}$ = 19.750

**Beispiel 11**

Unter den in Beispiel 3 angegebenen Bedingungen erhält man aus 1,8 g 11β-Cyclopropyl-3,3-(2,2-dimethyltrimethylendioxy)-5α-hydroxy-15β,16β-methylen-19-nor-17α-pregn-9-en-21,17-carbolacton 720 mg 11β-Cyclopropyl-15β,16β-methylen-3-oxo-19-nor-17α-pregna-4,9-dien-21,17-carbolacton.
IR: 1760, 1660 $cm^{-1}$
UV: $\varepsilon_{302}$ = 20.450

**Beispiel 12**

a) Eine Lösung von 1,7 g 3,3-(2,2-Dimethyltrimethylendioxy)-15β,16β-methylen-17α-(3-hydroxypropyl)-11β-vinyl-estr-9-en-5α,17β-diol in 15 ml Pyridin wird mit 1,2 g p-Toluolsulfonsäurechlorid versetzt und 16 Stunden bei Raumtemperatur gerührt. Anschließend verdünnt man mit Dichlormethan, wäscht mit Wasser und engt im Vakuum ein. Das erhaltene Rohprodukt wird durch Säulenchromatographie an Kieselgel gereinigt. Man erhält 1,35 g 3,3-(2,2-Dimethyltrimethylendioxy)-5α-hydroxy-15β,16β-methylen-11β-vinyl-östr-9-en-[17(β-1')-spiro-5']-perhydrofuran.

b) Unter den in Beispiel 3 a) beschriebenen Bedingungen erhält man aus 1,25 g 3,3-(2,2-Dimethyltrimethylendioxy)-5α-hydroxy-15β,16β-methylen-11β-vinyl-östr-9-en-[17(β-1')-spiro-5']-perhydrofuran 775 mg 15β,16β-Methylen-3-oxo-11β-vinyl-östra-4,9-dien-[17(β-1')-spiro-5']-perhydrofuran.

**Beispiel 13**

a) Unter den in Vorschrift 3 beschriebenen Bedingungen erhält man aus 12 g 3,3-(2,2-Dimethyltrimethylendioxy)-5α,10α-epoxy-15β,16β-methylen-17α-(3-hydroxypropinyl)-estr-9(11)-en-17β-ol 8,4 g 3,3-(2,2-Dimethyltrimethylendioxy)-17α-(3-hydroxypropinyl)-15β,16β-methylen-11β-vinyl-estr-9-en-5α,17β-diol.

b) Unter den in Beispiel 3 a) beschriebenen Bedingungen erhält man aus 8,3 g 3,3-(2,2-Dimethyltrimethylendioxy)-17α-(3-hydroxypropinyl)-15β,16β-methylen-11β-vinyl-estr-9-en-5α-17β-diol 6,2 g 17β-Hydroxy-17α-(3-hydroxypropinyl)-15β,16β-methylen-11β-vinyl-estra-4,9-dien-3-on.

c) Eine Lösung von 6,1 g 17β-Hydroxy-17α-(3-hydroxypropinyl)-15β,16β-methylen-11β-vinyl-estra-4,9-dien-3-on in 120 ml THF wird mit 3 g Lindlar-Katalysator hydriert. Nach Aufnahme von einem Äquivalent Wasserstoff wird vom Katalysator abfiltriert, im Vakuum eingeengt und das erhaltene Rohprodukt an Kieselgel chromatographiert. Man erhält 4,9 g 17β-Hydroxy-17α-(3-hydroxypropenyl)-15β,16β-methylen-11β-vinyl-estra-4,9-dien-3-on.

**Beispiel 14**

Unter den in Beispiel 3 b) beschriebenen Bedingungen erhält man aus 2 g 17β-Hydroxy-17α-(3-hydroxypropenyl)-15β,16β-methylen-11β-vinyl-estra-4,9-dien-3-on 1,15 g 15β,16β-Methylen-3-oxo-11β-vinyl-19-nor-17α-pregna-4,9,20-trien-21,17-carbolacton.
IR: 1735, 1660 $cm^{-1}$

**Beispiel 15**

Unter den in Beispiel 12 a) beschriebenen Bedingungen erhält man aus 2 g 17β-Hydroxy-17α-(3-hydroxypropenyl)-15β,16β-methylen-11β-vinyl-estra-4,9-dien-3-on 1,54 g 15β,16β-Methylen-3-oxo-11β-vinyl-estra-4,9-dien-[17(β-1')-spiro-5']-1',2'-dihydrofuran.

**Beispiel 16**

Eine Lösung von 500 mg 15β,16β-Methylen-3-oxo-11β-vinyl-19-nor-17α-pregna-4,9-dien-21,17-carbolacton in 10 ml THF wird mit 100 mg Lithiumaluminiumhydrid versetzt und 2 Stunden bei Raumtemperatur gerührt. Anschließend verdünnt man mit Essigester, wäscht mit verdünnter Schwefelsäure und Wasser und engt im Vakuum ein. Das erhaltene Rohprodukt wird in 25 ml $CH_2Cl_2$ aufgenommen und mit 5 g Mangandioxid über Nacht gerührt. Nach dem Abfiltrieren des Mangandioxids wird das erhaltene Rohprodukt an Kieselgel chromatographiert. Man erhält 285 mg 17β-Hydroxy-17α-(3-hydroxypropyl)-15β,16β-methylen-11β-vinyl-estra-4,9-dien-3-on.

**Patentansprüche** für die Vertragsstaaten DE, CH, LI, DE, FR, GB, IT, LU, NL, SE

1. 17-Substituierte Estradiene der allgemeinen Formel I

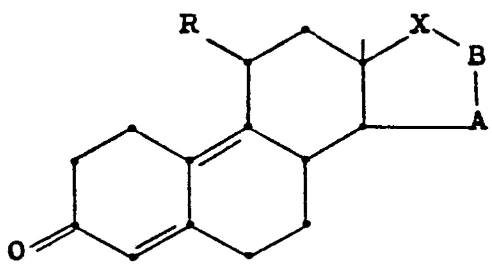 (I),

worin

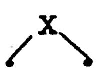 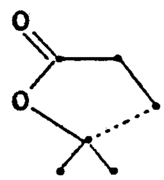, 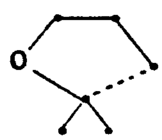, 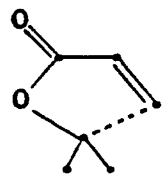, 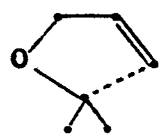,

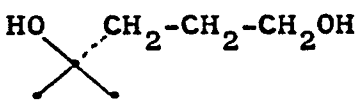 oder 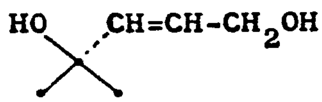,

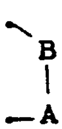 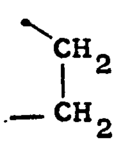 oder 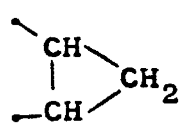

und

R Alkyl, Alkenyl oder Cycloalkyl mit bis zu 5 Kohlenstoffatomen,

darstellen

2. 17β-Hydroxy-17α-(3-hydroxypropyl)-11β-vinyl-4,9-estradien-3-on und 19-Nor-3-oxo-11β-vinyl-17α-pregna-4,9-dien-21,17-carbolacton.

3. 17β-Hydroxy-17α-(3-hydroxypropyl)-11β-isopropenyl-4,9-estradien-3-on und 19-Nor-3-oxo-11β-isopropenyl-17α-pregna-4,9-dien-21,17-carbolacton.

4. 17β-Hydroxy-17α-(3-hydroxypropyl)-11β-methyl-4,9-estradien-3-on und 11β-Methyl-19-nor-3-oxo-17α-pregna-4,9-dien-21,17-carbolacton.

5. 11β-Ethyl-17β-hydroxy-17α-(3-hydroxypropyl)-4,9-estradien-3-on und 11β-Ethyl-19-nor-3-oxo-17α-pregna-4,9-dien-21,17-carbolacton.

6. 17β-Hydroxy-17α-(3-hydroxypropyl)-11β-propyl-4,9-estradien-3-on und 19-Nor-11β-propyl-3-oxo-17α-pregna-4,9-dien-21,17-carbolacton.

7. 11β-Cyclopropyl-17β-hydroxy-17α-(3-hydroxypropyl)-4,9-estradien-3-on und 11β-Cyclopropyl-19-nor-3-oxo-17α-pregna-4,9-dien-21,17-carbolacton.

8. 11β-Cyclopentyl-17β-hydroxy-17α-(3-hydroxypropyl)-4,9-estradien-3-on und 11β-Cyclopentyl-17β-hydroxy-19-nor-3-oxo-17α-pregna-4,9-dien-21,17-carbolacton.

9. 11β-Methyl-15β,16β-methylen-3-oxo-19-nor-17α-pregna-4,9-dien-21,17-carbolacton, 15β,16β-Methylen-3-oxo-11β-vinyl-19-nor-17α-pregna-4,9-dien-21,17-carbolacton und 11β-Cyclopropyl-15β,16β-methylen-3-oxo-19-nor-17α-pregna-4,9-dien-21,17-carbolacton,

10. 15β,16β-Methylen-3-oxo-11β-vinyl-4,9-estradien-[17(β-1')-spiro-5']-perhydrofuran, 17β-Hydroxy-17α-(3-hydroxypropenyl)-15β,16β-methylen-11β-vinyl-4,9-estradien-3-on, 15β,16β-Methylen-3-oxo-11β-vinyl-19-nor-17α-pregna-4,9,20-trien-21,17-carbolacton, 15β,16β-Methylen-3-oxo-11β-vinyl-4,9-estradien-[17(β-1')-spiro-5']-1'2'-dihydrofuran und 17β-Hydroxy-17α-(3-hydroxypropyl)-15β,16β-methylen-11β-vinyl-4,9-estradien-3-on.

11. 11β-Cyclopropyl-17β-hydroxy-17α-(3-hydroxy-1-Z-propenyl)-4,9-estradien-3-on.

12. Pharmazeutische Präparate, gekennzeichnet durch den Gehalt an Verbindungen gemäß Anspruch 1 bis 11.

13. Verfahren zur Herstellung von 17-substituierten Estradienen der allgemeinen Formel I

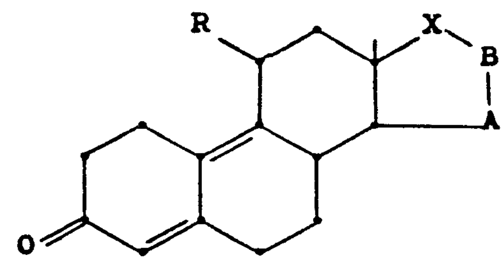

(I),

worin

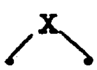 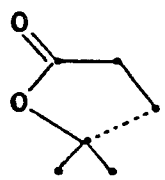, 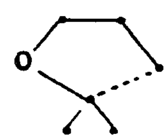, 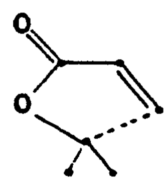, 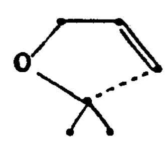,

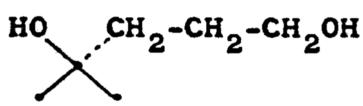 oder 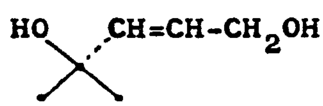,

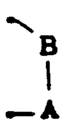 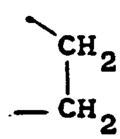 oder 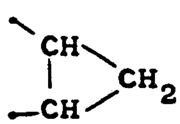 ;

und

R Alkyl, Alkenyl oder Cycloalkyl mit bis zu 5 Kohlenstoffatomen

darstellen,
dadurch gekennzeichnet, daß man in an sich bekannter Weise

a) eine Verbindung der allgemeinen Formel II

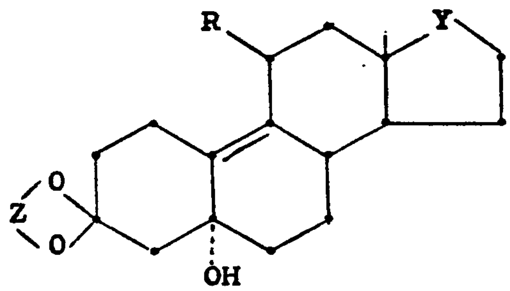

(II),

worin
R die in Formel I angegebene Bedeutung hat,
Y für X steht, wobei vorhandene freie Hydroxygruppen mit einer sauer leicht abspaltbaren Gruppe verethert sein können und
Z eine Ethylen- oder 2,2-Dimethylpropylengruppe
darstellt,
zur Ketalspaltung, Entfernung einer gegebenenfalls vorhandenen mit Säure abspaltbaren Schutzgruppe und gleichzeitigen Wasserabspaltung unter Ausbildung des 4,9-Dien-3-on-Systems mit verdünnter Säure, dem Pyridiniumsalz einer starken Säure oder einem sauren Ionenaustauscher behandelt und gegebenenfalls die so erhaltene 17α-(3-Hydroxypropyl)- oder 17α-(3-Hydroxypropenyl)-Verbindung zur 17-Spiro-Hydrofuran-Verbindung cyclisiert oder zum 21,17-Carbolacton oxydiert oder

b) eine Verbindung der allgemeinen Formel III

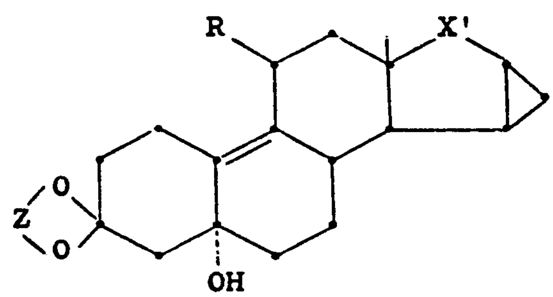

(III),

worin
R die in Formel I angegebene Bedeutung hat,
X'

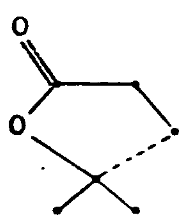, 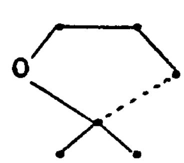, 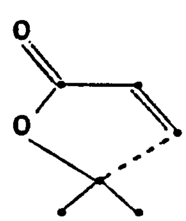 oder 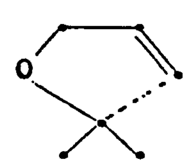

und
Z eine Ethylen- oder 2,2-Dimethylpropylengruppe
darstellen,
zur Ketalspaltung und gleichzeitigen Wasserabspaltung unter Ausbildung des 4,9-Dien-3-on-Systems mit verdünnter Säure, dem Pyridiniumsalz einer starken Säure oder einem sauren Ionenaustauscher behandelt und gegebenenfalls das so erhaltene 21,17-Carbolacton zur 17α-(3-Hydroxypropyl)- oder 17α-(3-Hydroxypropenyl)-Verbindung reduziert, die mitreduzierte 3-Ketogruppe mit Mangandioxid reoxydiert oder

c) eine Verbindung der allgemeinen Formel IV

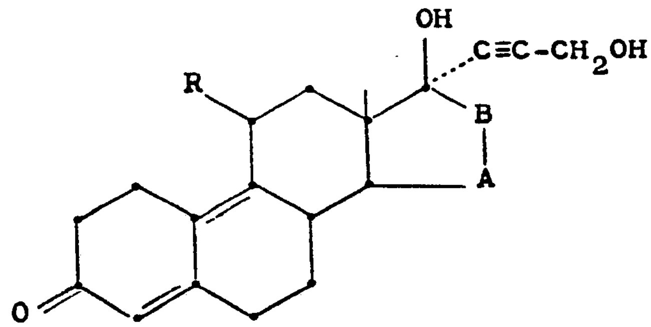

(IV),

worin B und R die in Formel I angegebene Bedeutung haben, zur 17α-(3-Hydroxypropenyl)-Verbindung
|
A

hydriert bzw. reduziert, diese gegebenenfalls zur entsprechenden 17-Spiro-Hydrofuran-Verbindung cyclisiert oder zum entsprechenden 21,17-Carbolacton oxydiert.

**Patentanspruch** für den Vertragsstaat AT

Verfahren zur Herstellung von 17-substituierten Estradienen der allgemeinen Formel I

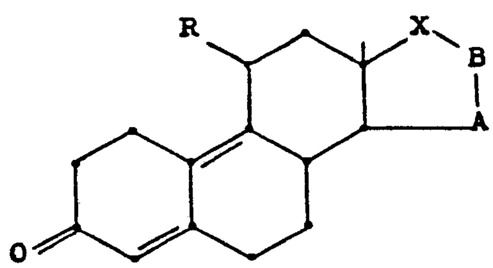

(I),

worin

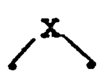 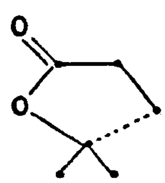, 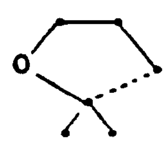, 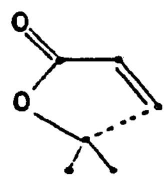, 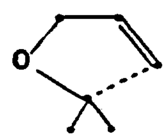,

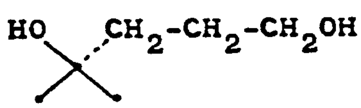 oder 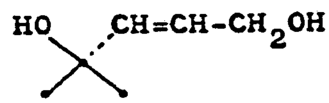

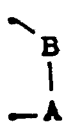 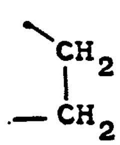 oder 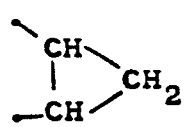 ;

und

R Alkyl, Alkenyl oder Cycloalkyl mit bis zu 5 Kohlenstoffatomen
darstellen,
dadurch gekennzeichnet, daß man in an sich bekannter Weise

a) eine Verbindung der allgemeinen Formel II

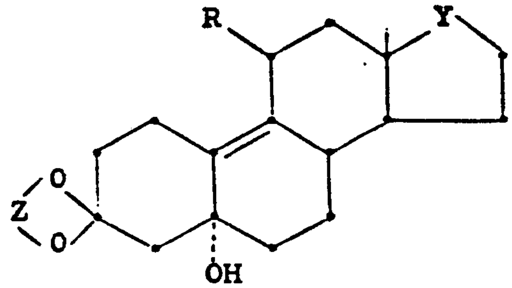

(II),

worin
R die in Formel I angegebene Bedeutung hat,
Y für X steht, wobei vorhandene freie Hydroxygruppen mit einer sauer leicht abspaltbaren Gruppe verethert sein können und
Z eine Ethylen- oder 2,2-Dimethylpropylengruppe
darstellt,
zur Ketalspaltung, Entfernung einer gegebenefalls vorhandenen mit Säure abspaltbaren Schutzgruppe und gleichzeitigen Wasserabspaltung unter Ausbildung des 4,9-Dien-3-on-Systems mit verdünnter Säure, dem Pyridiniumsalz einer starken Säure oder einem sauren Ionenaustauscher behandelt und gegebenefalls die so erhaltene 17α-(3-Hydroxypropyl)- oder 17α-(3-Hydroxypropenyl)-Verbindung zur 17-Spiro-Hydrofuran-Verbindung cyclisiert oder zum 21,17-Carbolacton oxydiert oder

b) eine Verbindung der allgemeinen Formel III

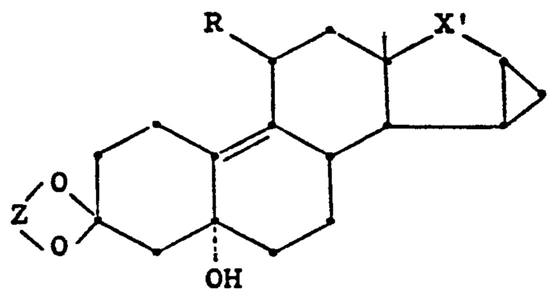

(III),

worin
R die in Formel I angegebene Bedeutung hat,
X'

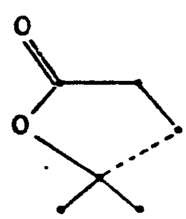
,
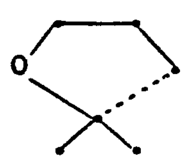
,
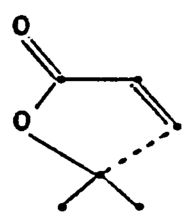
oder
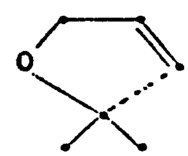

und
Z eine Ethylen- oder 2,2-Dimethylpropylengruppe
darstellen,
zur Ketalspaltung und gleichzeitigen Wasserabspaltung unter Ausbildung des 4,9-Dien-3-on-Systems mit verdünnter Säure, dem Pyridiniumsalz einer starken Säure oder einem sauren Ionenaustauscher behandelt und gegebenenfalls das so erhaltene 21,17-Carbolacton zur 17α-(3-Hydroxypropyl)- oder 17α-(3-Hydroxypropenyl)-Verbindung reduziert, die mitreduzierte 3-Ketogruppe mit Mangandioxid reoxydiert und oder

c) eine Verbindung der allgemeinen Formel IV

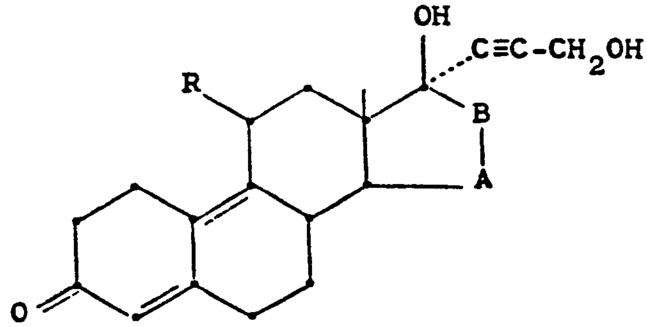

(IV),

worin B–A und R die in Formel I angegebene Bedeutung haben, zur 17α-(3-Hydroxypropenyl)-Verbindung hydriert bzw. reduziert, diese gegebenenfalls zur entsprechenden 17-Spiro-Hydrofuran-Verbindung cyclisiert oder zum entsprechenden 21,17-Carbolacton oxydiert.

**Claims** for Contracting States BE, CH, LI, DE, FR, GB, IT, LU, NL, SE

1. 17-substituted estradienes of general formula I

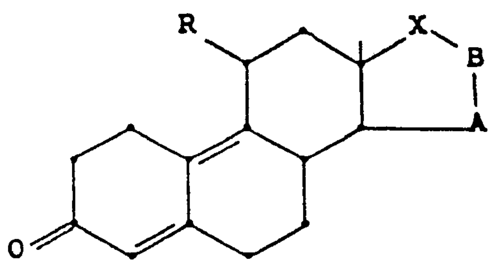

(I)

wherein

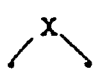 is 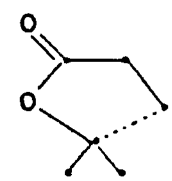, 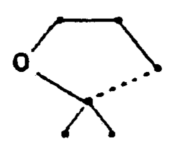, 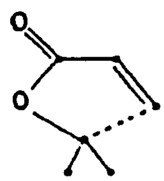, 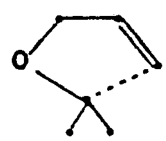,

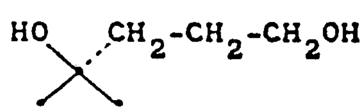 or 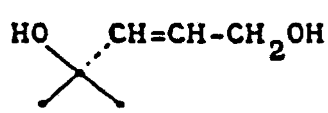,

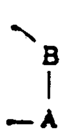 is 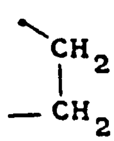 or 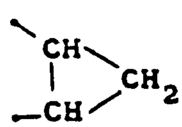

and
R is alkyl, alkenyl or cycloalkyl having up to 5 carbon atoms.

2. 17β-hydroxy-17α-(3-hydroxypropyl)-11β-vinyl-4,9-estradien-3-one and 19-nor-3-oxo-11β-vinyl-17α-pregna-4,9-diene-21,17-carbolactone.

3. 17β-hydroxy-17α-(3-hydroxypropyl)-11β-isopropenyl-4,9-estradien-3-one and 19-nor-3-oxo-11β-isopropenyl-17α-pregna-4,9-diene-21,17-carbolactone.

4. 17β-hydroxy-17α-(3-hydroxypropyl)-11β-methyl-4,9-estradien-3-one and 11β-methyl-19-nor-3-oxo-17α-pregna-4,9-diene-21,17-carbolactone.

5. 11β-ethyl-17β-hydroxy-17α-(3-hydroxypropyl)-4,9-estradien-3-one and 11β-ethyl-19-nor-3-oxo-17α-pregna-4,9-diene-21,17-carbolactone.

6. 17β-hydroxy-17α-(3-hydroxypropyl)-11β-propyl-4,9-estradien-3-one and 19-nor-11β-propyl-3-oxo-17α-pregna-4,9-diene-21,17-carbolactone.

7. 11β-cyclopropyl-17β-hydroxy-17α-(3-hydroxypropyl)-4,9-estradien-3-one and 11β-cyclopropyl-19-nor-3-oxo-17α-pregna-4,9-diene-21,17-carbolactone.

8. 11β-cyclopentyl-17β-hydroxy-17α-(3-hydroxypropyl)-4,9-estradien-3-one and 11β-cyclopentyl-17β-hydroxy-19-nor-3-oxo-17α-pregna-4,9-diene-21,17-carbolactone.

9. 11β-methyl-15β,16β-methylene-3-oxo-19-nor-17α-pregna-4,9-diene-21,17-carbolactone, 15β,16β-methylene-3-oxo-11β-vinyl-19-nor-17α-pregna-4,9-diene-21,17-carbolactone and 11β-cyclopropyl-15β,16β-methylene-3-oxo-19-nor-17α-pregna-4,9-diene-21,17-carbolactone.

10. 15β,16β-methylene-3-oxo-11β-vinyl-4,9-estradiene-[17(β-1')-spiro-5']perhydrofuran, 17β-hydroxy-17α-(3-hydroxypropenyl)-15β,16β-methylene-11β-vinyl-4,9-estradien-3-one, 15β,16β-methylene-3-oxo-11β-vinyl-19-nor-17α-pregna-4,9,20-triene-21,17-carbolactone, 15β,16β-methylene-3-oxo-11β-vinyl-4,9-estradiene-[17(β-1')-spiro-5']-1',2'-dihydrofuran and 17β-hydroxy-17α-(3-hydroxypropyl)-15β,16β-methylene-11β-vinyl-4,9-estradien-3-one.

11. 11β-cyclopropyl-17β-hydroxy-17α-(3-hydroxy-1-Z-propenyl)-4,9-estradien-3-one.

12. Pharmaceutical preparations, characterized in that they contain compounds according to claims 1 to 11.

13. Process for the preparation of 17-substituted estradienes of general formula I

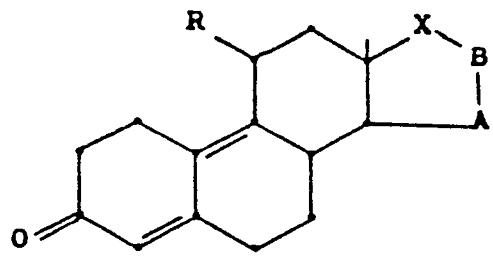

(I)

wherein

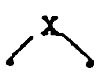 is 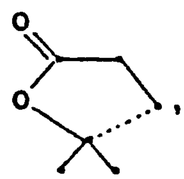, 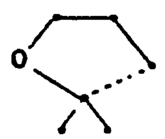, 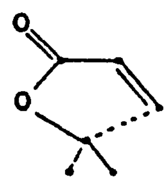, 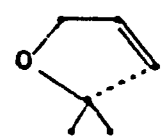,

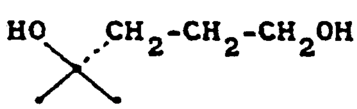 or 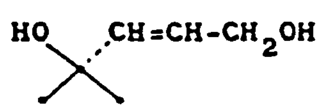,

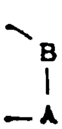 is 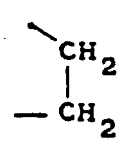 or 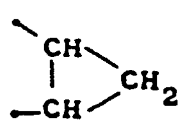

and
R is alkyl, alkenyl or cycloalkyl having up to 5 carbon atoms, characterized in that, in a manner known per se,

a) a compound of general formula II

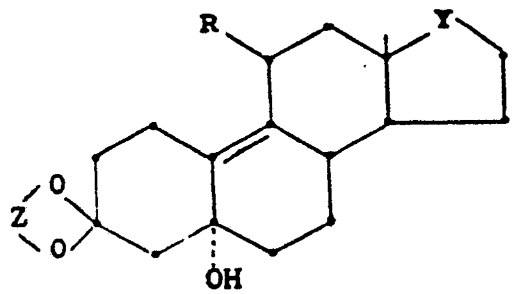

(II)

wherein

R has the meanings given in formula I,
Y stands for X wherein any free hydroxy groups present can be etherified with a group that can be readily split off under acidic conditions and
Z is an ethylene or 2,2-dimethylpropylene group,

is treated, for the purpose of ketal cleavage, removal of any protecting group that may be present and that can be split off with an acid and for simultaneously splitting off water with the formation of the 4,9-dien-3-one system, with a dilute acid, with the pyridinium salt of a strong acid or with an acidic ion exchanger, and optionally the resulting 17α-(3-hydroxypropyl) or 17α-(3-hydroxypropenyl) compound is cyclized to the 17-spiro-hydrofuran compound or oxidized to the 21,17-carbolactone, or

b) a compound of general formula III

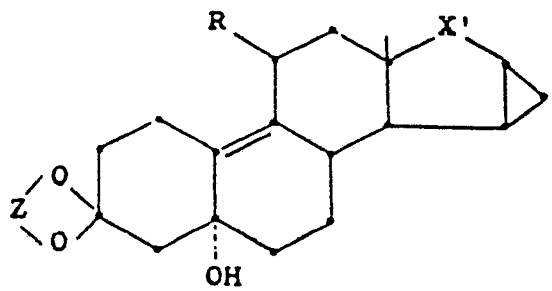

(III),

wherein

R has the meanings given in formula I,
X' is

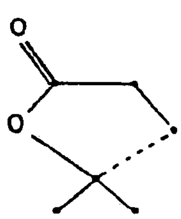, 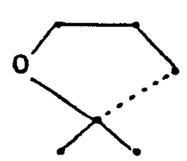, 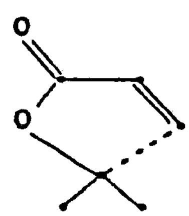 or 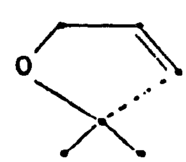

and
Z is an ethylene or 2,2-dimethylpropylene group,

is treated, for the purpose of ketal cleavage and simultaneously splitting off water with the formation of the 4,9-dien-3-one system, with a dilute acid, with the pyridinium salt of a strong acid or with an acidic ion exchanger, and optionally the resulting 21,17-carbolactone is reduced to the 17α-(3-hydroxypropyl) or 17α-(3-hydroxypropenyl) compound, and the concomitantly reduced 3-keto group is reoxidized with manganese dioxide, or

c) a compound of general formula IV

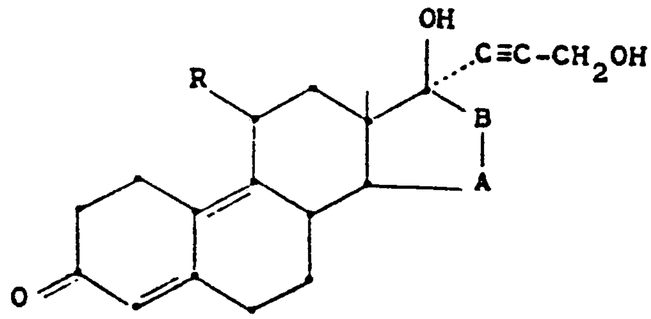

(IV)

wherein B—A and R have the meanings given in formula I, is hydrogenated or reduced to the 17α-(3-hydroxypropenyl) compound, and the latter is optionally cyclized to the corresponding 17-spiro-hydrofuran compound or oxidized to the corresponding 21,17-carbolactone.

**Claim** for Contracting State AT

Process for the preparation of 17-substituted estradienes of general formula I

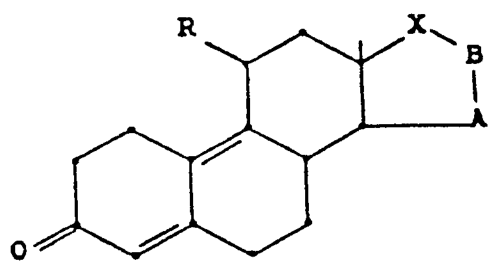

(I)

wherein

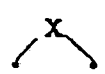 is 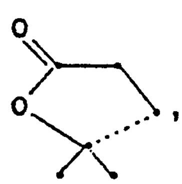, 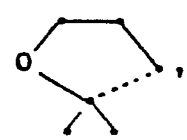, 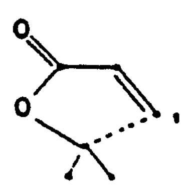, 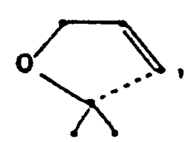,

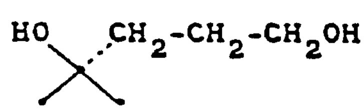 or 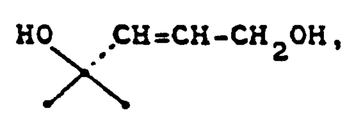,

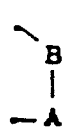 is 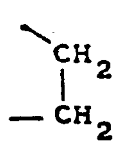 or 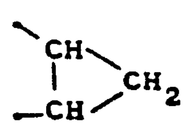

and

R is alkyl, alkenyl or cycloalkyl having up to 5 carbon atoms,

characterized in that, in a manner known <u>per se</u>,

a) a compound of general formula II

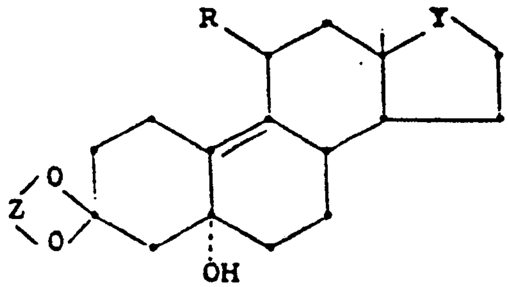

(II)

wherein

R has the meanings given in formula I,
Y stands for X wherein any free hydroxy groups present can be etherified with a group that can be readily split off under acidic conditions and
Z is an ethylene or 2,2-dimethylpropylene group,

is treated, for the purpose of ketal cleavage, removal of any protecting group that may be present and that can be split off with an acid and for simultaneously splitting off water with the formation of the 4,9-dien-3-one system, with a dilute acid, with the pyridinium salt of a strong acid or with an acidic ion exchanger, and optionally the resulting 17α-(3-hydroxypropyl) or 17α-(3-hydroxypropenyl) compound is cyclized to the 17-spiro-hydrofuran compound or oxidized to the 21,17-carbolactone, or

b) a compound of general formula III

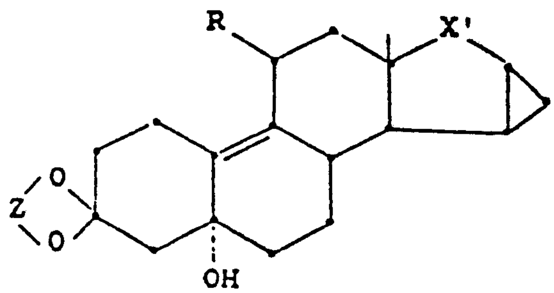

(III),

wherein

R has the meanings given in formula I,
X' is

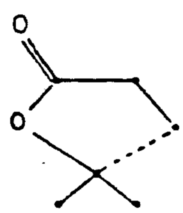 , 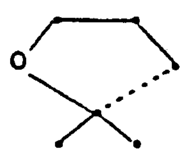 , 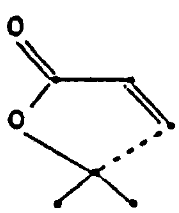 or 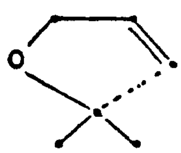

and
Z is an ethylene or 2,2-dimethylpropylene gruop,

is treated, for the purpose of ketal cleavage and simultaneously splitting off water with the formation of the 4,9-dien-3-one system, with a dilute acid, with the pyridinium salt of a strong acid or with an acidic ion exchanger, and optionally the resulting 21,17-carbolactone is reduced to the 17α-(3-hydroxypropyl) or 17α-(3-hydroxypropenyl) compound, and the concomitantly reduced 3-keto group is reoxidized with manganese dioxide, or

c) a compound of general formula IV

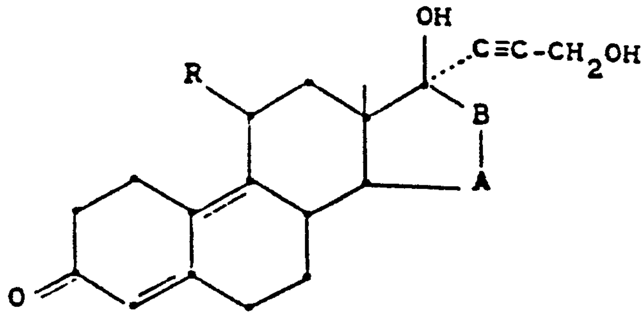

(IV)

wherein B and R have the meanings given in formula I, is hydrogenated or reduced to the 17α-(3-
|
A

hydroxypropenyl) compound, and the latter is optionally cyclized to the corresponding 17-spiro-hydrofuran compound or oxidized to the corresponding 21,17-carbolactone.

**Revendications** pour les Etats contractants BE, CH, DE, FR, GB, IT, LI, LU, NL, SE

1. Estradiènes substitués en 17 qui répondent à la formule générale I:

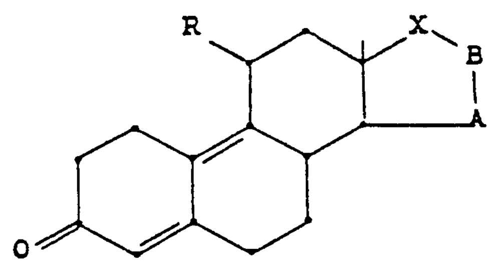

(I),

dans laquelle:

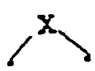 représente un radical répondant à l'une des formules:

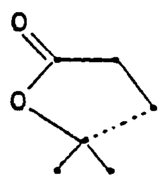, 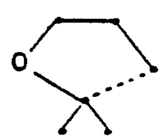, 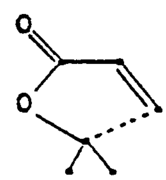, 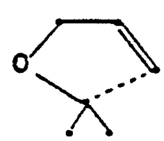,

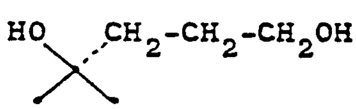 et 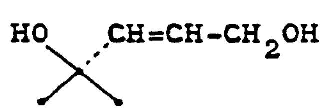,

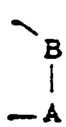 représente 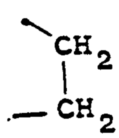 ou 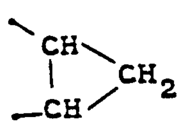 et

R représente un radical alkyle, alcényle ou cycloalkyle à au plus 5 atomes de carbone.

2. Hydroxy-17β (hydroxy-3 propyl)-17α vinyl-11β estradiène-4,9 one-3 et nor-19 oxo-3 vinyl-11β 17α-prégnadiène-4,9 carbolactone-21,17.

3. Hydroxy-17β (hydroxy-3 propyl)-17α isopropényl-11β estradiène-4,9 one-3 et nor-19 oxo-3 isopropényl-11β 17α-prégnadiène-4,9 carbolactone-21,17.

4. Hydroxy-17β (hydroxy-3 propyl)-17α méthyl-11β estradiène-4,9 one-3 et méthyl-11β nor-19 oxo-3 17α-prégnadiène-4,9 carbolactone-21,17.

5. Ethyl-11β hydroxy-17β (hydroxy-3 propyl)-17α estradiène-4,9 one-3 et éthyl-11β nor-19 oxo-3 17α-prégnadiène-4,9 carbolactone-21,17.

6. Hydroxy-17β (hydroxy-3 propyl)-17α propyl-11β estradiène-4,9 one-3 et nor-19 propyl-11β oxo-3 17α-prégnadiène-4,9 carbolactone-21,17.

7. Cyclopropyl-11β hydroxy-17β (hydroxy-3 propyl)-17α estradiène-4,9 one-3 et cyclopropyl-11β nor-19 oxo-3 17α-prégnadiène-4,9 carbolactone-21,17.

8. Cyclopentyl-11β hydroxy-17β (hydroxy-3 propyl)-17α estradiène-4,9 one-3 et cyclopentyl-11β hydroxy-17β nor-19 oxo-3 17α-prégnadiène-4,9 carbolactone-21,17.

9. Méthyl-11β méthylène-15β,16β oxo-3 nor-19 17α-prégnadiène-4,9 carbolactone-21,17, méthylène-15β,16β oxo-3 vinyl-11β nor-19 17α-prégnadiène-4,9 carbolactone-21,17 et cyclopropyl-11β méthylène-15β,16β oxo-3 nor-19 17α-prégnadiène-4,9 carbolactone-21,17.

10. Méthylène-15β,16β oxo-3 vinyl-11β estradiène-4,9 [17(β-1')-spiro-5']-perhydrofuranne, hydroxy-17β (hydroxy-3 propényl)-17α méthylène-15β,16β vinyl-11β estradiène-4,9 one-3 méthylène-15β,16β oxo-3 vinyl-11β nor-19 17α-prégnatriène-4,9,20 carbolactone-21,17, méthylène-15β,16β oxo-3 vinyl-11β estradiène-4,9 [17(β-1')-spiro-5']-(dihydro-1',2' furanne) et hydroxy-17β (hydroxy-3 propyl)-17α méthylène-15β,16β vinyl-11β estradiène-4,9 one-3.

11. Cyclopropyl-11β hydroxy-17β (hydroxy-3 propène-1-(Z) yl)-17α estradiène-4,9 one-3.

12. Compositions pharmaceutiques caractérisées en ce qu'elles contiennent des composés selon l'une quelconque des revendications 1 à 11.

13. Procédé pour préparer des estradiènes substitués en 17 qui répondent à la formule générale I:

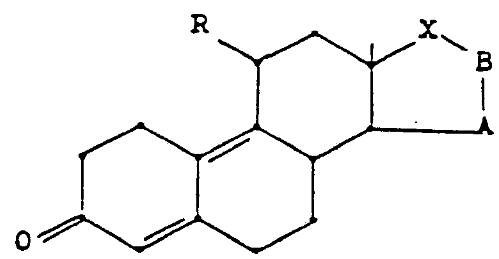

(I)

dans laquelle:

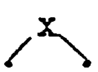 représente un radical répondant à l'une des formules:

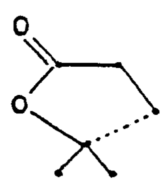, 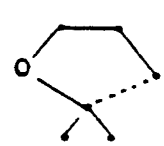, 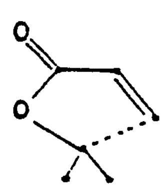, 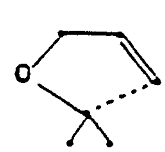,

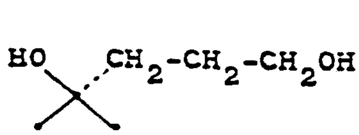 et 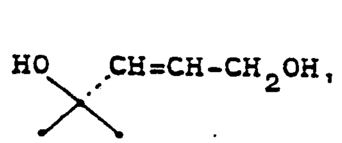

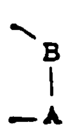 représente

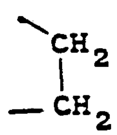 ou 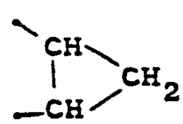 et

R représente un radical alkyle, alcenyle ou cycloalkyle contenant au maximum 5 atomes de carbone,

procédé caractérisé en ce que, en opérant de manière connue:

a) on traite par un acide dilué, par le sel de pyridinium d'un acide fort ou par un échangeur d'ions acide un composé répondant à la formule générale II:

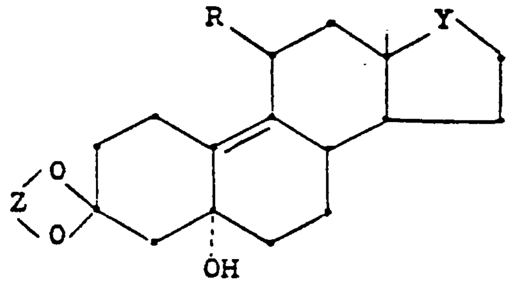

(II),

dans laquelle

R a la signification qui lui a été donnée à propos de la formule I,
Y représente l'un des radicaux mentionnés pour X, les éventuels radicaux hydroxy libres pouvant être éthérifiés par un radical facilement éliminable au moyen d'un acide, et
Z représente un radical éthylène ou un radical diméthyl-2,2 propylène,

de manière à couper le radical acétal, à éliminer un éventuel radical protecteur éliminable par un acide et, en même temps, à enlever de l'eau, pour provoquer la formation du système diène-4,9 one-3, et on cyclise éventuellement le composé ainsi obtenu qui porte un radical hydroxy-3 propyle ou hydroxy-3 propényle en 17α pour le convertir en le spiro-17 hydrofuranne, ou on l'oxyde pour le convertir en la carbolactone-21,17, ou

b) on traite par un acide dilué, par le sel de pyridinium d'un acide fort ou par un échangeur d'ions acide un composé répondant à la formule générale III:

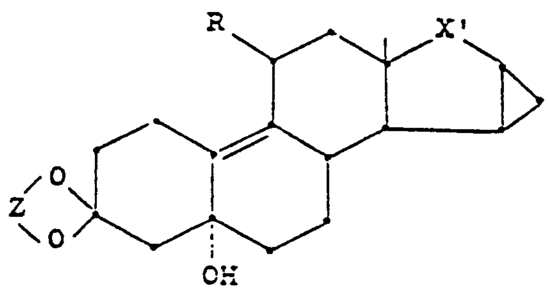

(III)

dans laquelle:

R a la signification qui lui a été donnée à propos de la formule I,
X' représente l'un des radicaux suivants:

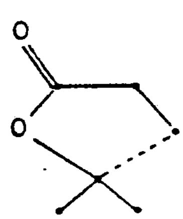 , 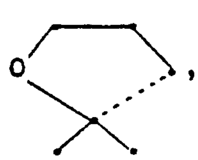 , 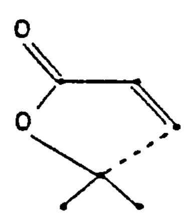 et 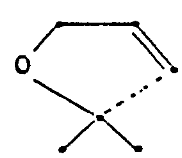

et
Z représente un radical éthylène ou un radical diméthyl-2,2 propylène,

de manière à couper le radical acétal et, en même temps, à enlever de l'eau, pour former le système diène-4,9 one-3, on réduit éventuellement la carbolactone-21,17 ainsi obtenue pour la transformer en le composé portant un radical hydroxy-3 propyle ou hydroxy-3 propényle en position 17α, et on réoxyde par du dioxyde de manganèse le radical oxo-3 qui a été réduit en même temps, ou

c) on hydrogène ou réduit un composé répondant à la formule générale IV:

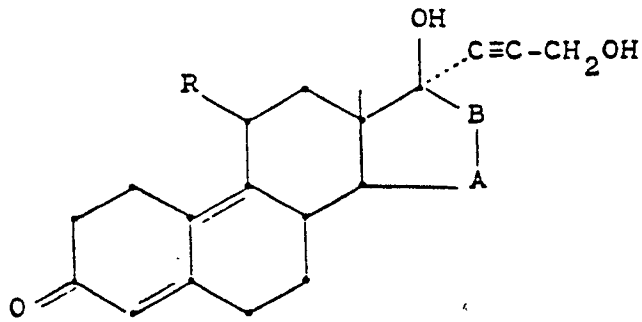

(IV)

dans laquelle $\begin{matrix}B\\|\\A\end{matrix}$ et R ont les significations qui leur ont été données à propos de la formule I, de manière à obtenir le composé portant un radical hydroxy-3 propényle en 17α, on cyclise éventuellement celui-ci pour obtenir le spiro-17 hydrofuranne correspondant ou on l'oxyde pour le transformer en la carbolactone-21,17 correspondante.

**Revendication** pour l'Etat contractant AT

Procédé pour préparer des estradiènes substitués en 17 qui répondent à la formule générale I:

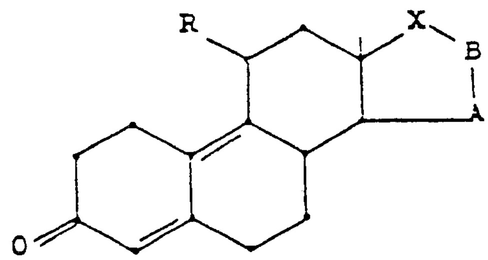

(I)

dans laquelle:

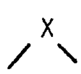 représente un radical répondant à l'une des formules:

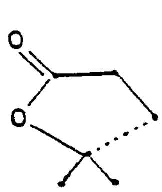 , 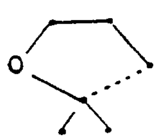 , 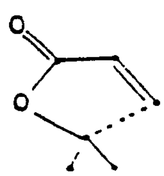 , 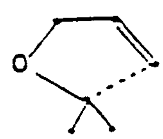 ,

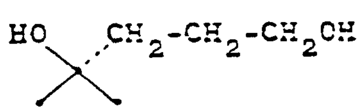 et 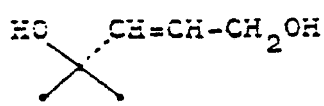 ,

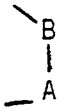 représente 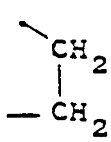 ou 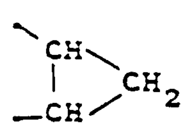 et et

R représente un radical alkyle, alcényle ou cycloalkyle à au plus 5 atomes de carbone,

procédé caractérisé en ce que, en opérant de manière connue:

a) on traite par un acide dilué, par le sel de pyridinium d'un répondant à la formule générale II:

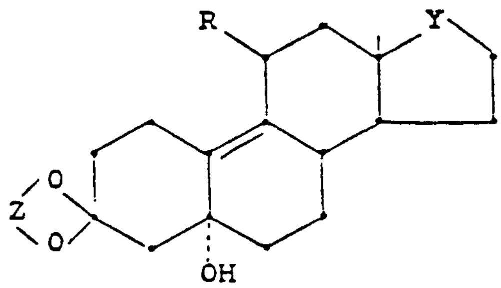

(II)

dans laquelle

R a la signification qui lui a été donnée à propos de la formule I,
Y représente l'un des radicaux mentionnés pour X, les éventuels radicaux hydroxy libres pouvant être éthérifiés par un radical facilement éliminable au moyen d'un acide, et
Z représente un radical éthylène ou un radical diméthyl-2,2 propylène,

de manière à couper le radical acétal, à éliminer un éventuel radical protecteur éliminable par un acide et, en même temps, à enlever de l'eau, pour provoquer la formation du système diène-4,9 one-3, et on cyclise éventuellement le composé ainsi obtenu qui porte un radical hydroxy-3 propyle ou hydroxy-3 propényle en 17α pour le convertir en le spiro-17 hydrofuranne, ou on l'oxyde pour le convertir en la carbolactone-21,17, ou

b) on traite par un acide dilué, par le sel de pyridinium d'un acide fort ou par un échangeur d'ions acide un composé répondant à la formule générale III:

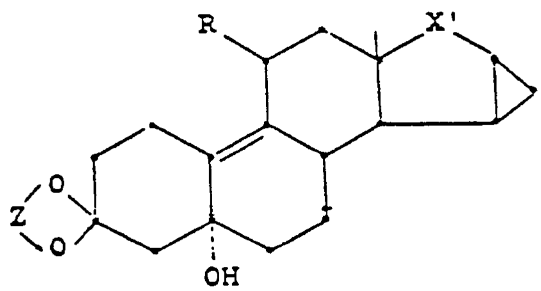

(III)

dans laquelle:

R a la signification qui lui a été donnée à propos de la formule I,
X' représente l'un des radicaux suivants

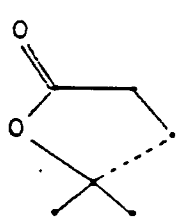 , 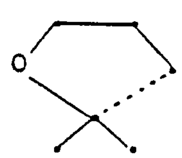 , 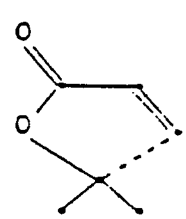 et 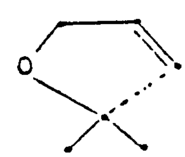

et
Z représente un radical éthylène ou un radical diméthyl-2,2 propylène,

de manière à couper le radical acétal et, en même temps, à enlever de l'eau, pour former le système diène-4,9 one-3, on réduit éventuellement la carbolactone-21,17 ainsi obtenue pour la transformer en le composé portant un radical hydroxy-3 propyle ou hydroxy-3 propényle en position 17α, et on réoxyde par du dioxyde de manganèse le radical oxo-3 qui a été réduit en même temps, ou

c) on hydrogène ou réduit un composé répondant à la formule générale IV:

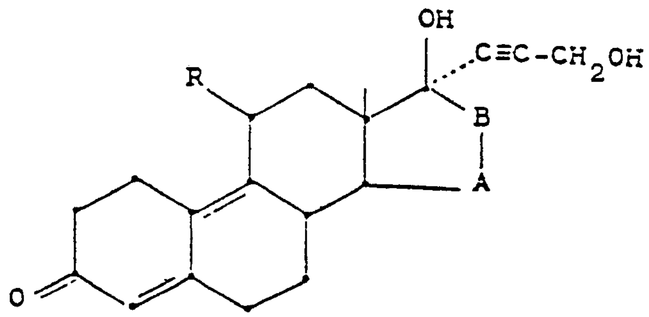

(IV)

dans laquelle B et R ont les significations qui leur ont été données à propos de la formule I, de manière à
|
A

obtenir le composé portant un radical hydroxy-3 propényle en 17α, on cyclise éventuellement celui-ci pour obtenir le spiro-17 hydrofuranne correspondant ou on l'oxyde pour le transformer en la carbolactone-21,17 correspondante.